# EUROPEAN PATENT APPLICATION

(11) **EP 3 147 580 A1**
(43) Date of publication of application: **29.03.2017**
(21) Application number: 15782814.6
(22) Date of filing: 10.04.2015
(51) Int. Cl.: F24F 3/16, A61L 9/16

(54) **ENVIRONMENT-IMPROVING AND HEALTHFUL AIR CONDITIONING DEVICE AND APPLICATION METHOD THEREOF**

(30) Priority: 23.04.2014 CN 201410163681
(71) Applicant: Fang, Moxi, Beijing 100086 (CN)
(72) Inventor: Fang, Moxi, Beijing 100086 (CN)
(74) Representative: Gassner, Birgitta
(86) International application number: PCT/CN2015/076292
(87) International publication number: WO 2015/161742

(57) **Abstract**

Disclosed is an air conditioning device, comprising an indoor unit (4), and an outdoor unit (5). The indoor unit (4) comprises an air guide grid (1) and an electron emitting assembly (2), and the electron emission assembly (2) includes an electron emitter (3).

## Description

### Field of the Invention

The present invention relates to an air conditioning device, and more particularly, to an air conditioning device capable of disinfecting a space and improving the immunity of organism and a method thereof, so as to improve the environment and strengthen the health of a human body.

### Background Art

At present, air conditioning devices used under various environments in the whole world (comprising the air conditioning devices and household air conditioners used in the environments including scientific research, industrial and agricultural production, and special works) have the main functions of adjusting the environmental temperature and properly adjusting the environmental humidity, and a filter screen attached in the air conditioner cannot filter the floating dust smaller than micrometer. Moreover, due to the reduction of ventilation and effects of temperature and humidity, the contaminant formed by virus, bacteria, and various factors in the environment not only cannot be eliminated, but also may cause the propagation of the bacteria and virus in the environment and the accumulation of the concentration of noxious substances, which is an important reason generating and spreading some diseases (that are also referred to "air-condition diseases" in some papers). The environment of using the air conditioning device above causes the accumulation of environmental pollution, which not only damages the health of a human body, but also is a major reason for the latency and generation of multiple diseases. At present, manufacturers in the whole world only make some technical improvements on the air conditioning device, such as the improvement from variable frequency technical technology and other improvements. The performances and effects of these air conditioning devices are not fundamentally reformed and improved; therefore, the production environment, working environment, and dwelling environment cannot be fundamentally improved, and this is the most important problem of the current air conditioning device.

The present invention is a healthful air conditioning device capable of improving the industrial and agricultural production environment, scientific research environment, the environments of various special works, public environment, and dwelling environment, so that the air conditioning device plays a more important role.

Although a patent entitled "Device and Method for Producing Negative Electric Nano Particles" refers that negative electric nano particles can be applied to multiple fields including household appliances, improvement of environmental state, the questions about how to introduce the negative electric nano particles into these fields and which devices or products the negative electric nano particles are introduced into are not mentioned. Certainly, it does not answer the questions about the effects of introducing the negative electric nano particles into these fields or devices, whether the problem of the current air conditioning devices in the world is solved after introducing a negative electric nano particle generator into the air conditioning device currently, and what fundamental major changes occurred to the performance and effect of the existing air conditioning device. Further, the question about how to specifically achieve the object on the parameter and structure is needed to be answered. At present, no data in the world refers to introducing the technology of the negative electric nano particles into the air conditioning device, and no data and practice can be used to answer the questions above. The questions above shall be answered depending on new research and practice.

The main structural parts of various air conditioning devices at current comprise a compressor, a compression motor, a heat exchanger, a capillary tube, an electronic control expansion valve, an electric heater, a four-way reversing valve, a fan motor, a master switch, a thermostatic controller, a defroster, a cold and hot switch, a heat protector, a frequency conversion fuzzy controller (minicomputer controller), a duct, a ventilation grid, a box body and/or a shell, etc. A central or concentrated air conditioning device is also provided with an air supply duct and an air supply outlet. The various air conditioning devices at current are composed of a refrigerating system and/or a heating system formed by the structural parts above, an electrical control system, an air circulation and/or conduction system and a box body and/or additional shell.

### Summary of invention

The present invention aims at providing a healthful air conditioning device capable of developing the air conditioning device that properly adjusts the temperature and humidity of the ambient temperature into the one having the effects of improving the environments of production, scientific research, and special works, and the public and dwelling environments at the same time. According to the present invention, the electron emission assembly is added into various air conditioning devices at current, and a negative electric nano particle generator is introduced into the air conditioning device.

According to an exemplary embodiment of the present invention, an environment-improving and healthful air conditioning device comprises a refrigerating system or a heating system or a system integrating the two, an electrical control system, an air circulation and/or conduction system, a box body and/or additional shell, and an electron emission assembly, wherein the electron emission assembly is arranged in various air conditioning devices fixedly configured in related environment or an air conditioning device in various portable equipment. The electron emission assembly configured for various air conditioning devices is corresponding to and matched with the part of each air supply outlet or scavenge port, or ventilation grid to form an entirety on structure or divide separately to become an independent part. The electron emission assembly is formed by connecting an electron emitter, an emission window, and various corresponding parts thereof to a related power supply and control part through a lead wire; the electron emitter is not only a part of the electron emission assembly, but also is a unique electrode having one potential relative to a ground potential outside the device, and only emits electrons to the space outside the device; the electrode comprises various metals, alloys, carbon materials, or composite materials, or other materials for emitting electrons; the electrode is in an acicular shape, a sharp shape, a threadlike shape, a clavate shape, a serrated shape, or a plate shape, and is designed into different structures and sizes according to the need; the electrode comprises a single or multiple electrode(s) with the same electric potential; and the electrode has different potentials ranging from -1 Kv to -35Kv relative to the ground potential outside the air conditioning device according to different materials, shapes, structures, sizes and requirements of the electrode. The electrons emitted by the electron emitter in the electron emission assembly are bound to oxygen molecules, carbon dioxide molecules, and water vapor molecules in air, or a molecular group composed of the molecules, or a nanoscale molecular group composed of the molecules with other molecules in air, so as to form negative electric nano particles distributed in corresponding air conditioning space corresponding electron emission assemblies are designed and distributed, and electron emitters with different quantities and the same potential are distributed according to the volume, shape, structure, size and requirement of the air conditioning space, and the structures and sizes of various air supply outlets, or scavenge ports, or ventilation grids, so that the density of the negative electric nano particles formed in the air conditioning space through the electrons emitted by the electron emitter is adjusted within a range more than 5 x 10³/cm³ but less than or equal to 10⁹/cm³ according to different use purposes and requirements, so that the corresponding air conditioning space is in an optimal health state. The electrons emitted by the electron emitter in the electron emission assembly form the negative electric nano particles with a certain density, the negative electric nano particles are distributed in the air conditioning space, and the air conditioning space is in a disinfection state; the negative electric nano particles in the air conditioning space can enter a human body through a respiratory system to provide lost electrons caused by the oxidative damage of DNA for the organism, so as to improve the immunity of organism and strengthen the health of a human body; the electrons and the negative electric nano particles formed by the electrons can quickly eliminate the damage to the human body caused by various contaminants, toxic materials, radioactive materials, and harmful nanoscale particles floating in air, so as to improve the dwelling environment and other special environments, and guarantee the successful performance of scientific research, industrial and agricultural production, and various works under special environment; and using the air conditioning device in various public places can prevent and control the spread of various respiratory system diseases and other diseases with infectiousness and can eliminate the harmful germs and harmful substances in the air of production greenhouse and livestock shed in the production of agriculture and livestock breeding, optimize the breed, prevent and control the occurrence and spread of various diseases, and improve the yield. Due to the configuration of the electron emission assembly, the refrigerating system and/or the heating system, and the corresponding parts thereof can be removed from the air conditioning device as long as the parts matched with the electron emission assembly are combined again, so that the structure of the device is changed, and the volume and size of the entire device are reduced; although no temperature regulation is configured, the air conditioning device is still an environment-improving and healthful air conditioning device that not only reduces the cost, but also can expand the application of the device better and more easily; due to the configuration of the electron emission assembly, the refrigerating system or the heating system of the air conditioning device with the refrigerating system and the heating system is closed in case that the ambient temperature is suitable, and the air volume is adjusted according to the need, so that the air conditioning device is still an environment-improving and healthful air conditioning device that not only saves the energy consumption, but also greatly extends the working time of the air conditioning device, and the air conditioning device may be used at any time all the year round according to the need, which preferably serves for improving the environment and being healthful.

Compared with the prior art, the present invention has the effective effects that: since the working region of the air conditioning device is full of the negative electric nano particles with a certain density at the same time, the performances and effects of the original air conditioning device are kept, and the following functions and effects are added meanwhile:
1. The negative electric nano particle is less than a bacteria and a virus, in a region covered by the negative electric nano particles with a certain density, the biotic environment of the bacteria and virus in the region is completely changed, and a plurality of negative electric nano particles directly acting on the bacteria and virus lead to the aberration of the bacteria and virus, and even lead to the fragmentation and breakup of the bacteria and virus, so as to lead to quick death of the bacteria and virus. The bacteria and virus are killed directly, so as to achieve the purpose of disinfecting the air environment.
2. In the space environment under the effect of the negative electric nano particles, a plurality of contaminants that are harmful to the health of a human body and float in air can be eliminated quickly.
   (1) Various harmful substances floating in the environmental air can be eliminated quickly, comprising various contaminants and toxicants floating in air;
   (2) Radioactive substances floating in the environmental air, such as radon and a daughter thereof in structural materials; and
   (3) Other nano particles that are harmful to the human body and float in the environmental air, especially the nano particles floating in the air in the nanometer research and production environments that will enter the human body through the respiratory system and esophagus to lead to a damage. At present, there is no method in the world that can eliminate the damage to the human body in such environment. However, this damage has limited various scientific researches capable of producing the nano particles and the industrial and agricultural production currently. Since a plurality of particles can be combined into a big particle after a plurality of collisions between the negative electric nano particles and the various harmful substances above, these big particles no longer float in the air and cannot enter the human body through the respiratory system, and the big particles subside quickly under the actions of a gravitational force and an electric field force, and the subsided particle dust may be removed through cleaning.
3. In the resent 50 years, biologists in the whole world have found that the free radical generated by the organism and other active byproducts generated by the metabolism and environment pollutants enter the organism, and mainly are active oxygen-containing materials, and these superoxides can lead to more than 100 different types of oxidative damages to the internal base pair of the DNA, i.e., to capture the electrons of DNA double helix structure. Researches show that each pair of chromosomes in the human body cell shall be attacked for 30,000 times every day. The body of an adult is averagely composed of 10 trillion cells. Therefore, the DNA of each person will repel approximately 300,000 trillion attacks every day, and all these attacks are possible to lead to the damage of the DNA. That is, each person may cause the electron loss of the DNA with a certain quantity in the organism every day. If the purine losing the electron in the DNA cannot be repaired timely, and react with water molecules under a certain condition, the base cytosine with normal pair in the DNA may form a wrong pair, i.e., transgenation. While the variation caused by base oxidation is an important reason that leads to the cancer, genetic disease, and necrocytosis.
   The researches of the biologist also show that the DNA double helix has the charge transfer function, and more than 130 types of human gene waves are verified and presumed to be related to the repair of the DNA, which prove that the human body has very strong self-repairing capability, and the electron plays an important role in the repair of the DNA. Although a lot of repair enzymes provide their electrons to fix the damaged place, it usually is insufficiency, and in some cases, it is very insufficiency, so as to lead to the occurrence of a plurality of diseases on human body. Therefore, the supplement of the electrons to the human body is useful and necessary. The negative electric nano particles formed by oxygen molecules, carbon dioxides, or vapor molecules carrying with the electrons in the air environment, or a molecular group composed of these molecules and other molecules enter the human body through the respiratory system and release the electrons thereof, so as to reach to the whole body through the blood circulation, supplement the lost electrons in the organism caused by the oxidative damage, improve the immunity of the organism, and strengthen the health of a human body.
4. The air conditioning device with the electron emission assembly has the following new effects, functions and applications at the same time.
   (1) The dwelling environment and other special work environments are improved, so that the crowd living or working in the environments are not harmed;
   (2) The spread of various respiratory system diseases and other diseases with infectiousness is prevented and controlled in various public places comprising various public traffics; and
   (3) The air conditioning device is designed into various special and dedicated air conditioning devices, and the air conditioning devices can adjust the density of the negative electric nano particles in the space within a range more than 5 x 10³/cm³ but less than or equal to 10⁹/cm³ according to different purposes and different requirements, so as to enable the corresponding air conditioning space to be in an optimal health state. Therefore, the requirements on the environments of various public places (comprising various public traffic systems), hospitals, and sanatoriums and the requirements on various mobile devices (comprising various vehicles, ships, aircrafts, aerospace equipment, etc.) and fixed equipment, scientific research, industrial and agricultural production, livestock shed, and various special environments are met, and the effects thereof lie in: ① being not only capable of eliminating the damage to the human body caused by toxic and harmful nano particles floating in air in the scientific research and agricultural production, so as to improve the environment and protect the health of the human body, but also guaranteeing the successful performance of various scientific researches, industrial and agricultural productions, and various works under special environment; and ② eliminating various harmful germs and harmful substances in the production greenhouse and livestock shed in the agricultural and livestock breeding productions; optimizing the breed; and preventing and controlling the occurrence and spread of various diseases, and being able to improve the yield. For example, in the production of various edible fungus, and under the action of the negative electric nano particles, various harmful germs can be eliminated, the breed is optimized, and the edible fungus grow flourishingly; and in the livestock shed, and under the action of the negative electric nano particles with a certain density, these sites are in the disinfection state, which prevents and controls the occurrence and spread of various transmissible diseases including the bird flu, etc. so that the chicken, cattle, and other fowls can grow healthily, and the yield of eggs and milk can be increased. At present, no material in the world refers to a device and/or a method that can achieve the comprehensive performances and effects above at the same time.

In conclusion, it can be further concluded that due to the configuration of the electron emission assembly, the refrigerating system and/or the heating system, i.e., the corresponding parts thereof can be removed from the air conditioning device as long as the parts matched with the electron emission assembly are combined again, so that the structure of the device is changed, and the volume and size of the entire device are reduced; although no temperature regulation is configured, the air conditioning device is still an environment-improving and healthful air conditioning device that not only reduces the cost, but also can expand the application of the device better and more easily; due to the configuration of the electron emission assembly, the refrigerating system or the heating system of the air conditioning device with the refrigerating system and the heating system is closed in case that the ambient temperature is suitable, and the air volume is adjusted according to the need, so that the air conditioning device is still an environment-improving and healthful air conditioning device that not only saves the energy consumption, but also greatly extends the working time of the air conditioning device, and the air conditioning device may be used at any time all the year round according to the need, which preferably serves for improving the environment and being healthful.

### Brief description of drawings

Fig. 1 is a perspective view illustrating an upright air conditioning device according to the invention;
Fig. 2 is a perspective view illustrating a concentrated or central air conditioning device according to the invention; and
Fig. 3 is a perspective view illustrating an outdoor unit according to the invention.

### Detailed description of embodiments

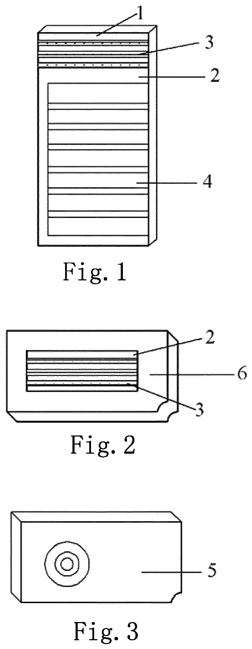

Fig. 1 is a perspective view illustrating an upright air conditioning device, and Fig. 3 is a perspective view illustrating an outdoor machine comprising an outdoor unit 5 (mainly composed of an axial fan, a compressor, a heat exchanger, a four-way reversing valve, a capillary tube ball valve, a control circuit, etc.) and a box body. The structures of an electron emission assembly 2 and an electron emitter 3 therein and the structure of a ventilation grid 1 may be arranged uniformly as shown in Fig. 1, or may be arranged separately to become independent parts respectively. Both of the two arrangements are based on the principle of satisfying respective functions and effects.

If the electron emission assembly 2 emits electrons based on tunneling effect, a power supply thereof only provides potential to the electron emitter. Comprising the losses of an electronic assembly and an electric circuit, the energy consumption has been lower than 3w under the existing technical condition, and the sizes of the circuit and the assembly thereof are smaller, which may not affect the size of the box body of an indoor unit 4, wherein the circuit thereof may be interacted and interlocked with other electrical parts of the air conditioning device, and meanwhile, corresponding independent control keys may be set on a control panel or a remote-operated controller.

Fig. 2 is a perspective view illustrating a concentrated or central air conditioning device of the invention. An air supply outlet 6 is combined with the electron emission assembly 2 to perform structural design and distribution, so as to form the air supply outlet 6 having the electron emission assembly 2; and the functions and effects of all rest parts and ducts of the concentrated or central air conditioning device may be unchanged. With regard to the control of the electron emission assembly 2, the concentrated or central air conditioning device may be interacted and interlocked with the control assembly through a lead wire according to different requirements, and corresponding independent control keys are arranged on the control panel or the remote-operated controller.

According to the examples as shown above, the electron emission assembly is only added on the basis of a commercially available air conditioning device at current, so as to briefly explain how to achieve the object of the invention. No matter the solution above is adopted, or each system, part, and box body and/or shell in the air conditioning device are recombined and improved to form various new and different or dedicated air conditioning devices, or the electron emission assembly is arranged in the air conditioning device that does not need temperature regulation, the object stated above of the corresponding space can be implemented by emitting electrons to the space in the corresponding range and making the density of the negative electric nano particles in the corresponding space be more than 5 x 10³/cm³ but less than or equal to 10⁹/cm³.

According to the invention, the electron emission assembly is added in various air conditioning devices at current. Various air conditioning devices comprise various air conditioning devices fixedly arranged in the air conditioning device of related environmental or various portable equipment; these air conditioning devices may work using a manner of the central air conditioner, or the concentrated air conditioner, or a single independent air conditioner, and all the air conditioning devices above are provided with corresponding electron emission assemblies.

The arrangement of the electron emission assemblies in various air conditioning devices is that the electron emission assembly 2 is corresponding to and matched with each of the parts of the air supply outlet or the scavenge port or the ventilation grid 1 to form an integral part in structure or to divide separately to become independent parts. The electron emission assembly 2, which is connected to a related power supply and control part through a lead wire, comprises an electron emitter, an emission window and various corresponding parts thereof; the electron emitter is not only a part of the electron emission assembly, but also a unique electrode having one potential relative to a ground potential outside the device, and it only emits electrons to the space outside the device; the electrode may be made of different materials (such as metal, alloy, carbon material, composite material, or other material for emitting electrons); the electrode may be composed of different shapes (such as an acicular shape, a sharp shape, a threadlike shape, a clavate shape, a serrated shape, or a plate shape); the electrode may be designed into different structures and sizes according to the need; the electrode may be composed of a single or multiple electrodes with the same electric potential; and the electrode has different potentials ranging from -1 Kv to -35Kv relative to the ground potential outside the air conditioning device according to different materials, shapes, structures, sizes and requirements of the electrode.

As present technology is concerned, researches show that a tunnel effect technology used to emit electrons in the electron emission assembly is the most suitable art applied in the air conditioning device. The consumption power of the electron emission assembly being lower than 3w is already implemented in the prior art. The electrons emitted by the electron emitter in the electron emission assembly are bound to oxygen molecules, carbon dioxide molecules, and water vapor molecules in air, or a molecular group composed of these molecules, or a nanoscale molecular group composed of these molecules with other molecules in air, so as to form negative electric nano particles distributed in corresponding air conditioner space; corresponding electron emission assemblies are designed and distributed, and electron emitters with different quantities and the same potential are distributed according to the volume, shape, structure, size and requirement of the air conditioning space, and the structures and sizes of various air supply outlets, or scavenge ports, or ventilation grids, so that the density of the negative electric nano particles formed in the air conditioning space through the electrons emitted by the electron emitter is adjusted within a range more than 5 x 10³/cm³ but less than or equal to 10⁹/cm³ according to different use purposes and requirements, so that the corresponding air conditioning space is in a disinfected state.

## Claims

1. An environment-improving and healthful air conditioning device, comprising a refrigerating system and/or a heating system, an electrical control system, an air circulation and/or conduction system, and a box body and/or additional shell, wherein the environment-improving and healthful air conditioning device further comprises an electron emission assembly that is configured to various air conditioning devices fixedly arranged in related environment or configured to an air conditioning device in various portable apparatus.

2. The environment-improving and healthful air conditioning device according to claim 1, wherein the electron emission assembly configured to various air conditioning devices is corresponding to and matched with each of the parts of air supply outlet or the scavenge port, or the ventilation grid to form an integral part in structure or to divide separately to become independent parts.

3. The environment-improving and healthful air conditioning device according to claim 1, wherein the electron emission assembly is formed by connecting an electron emitter, an emission window, and various corresponding parts thereof to a related power supply and control part through wires;
the electron emitter is not only a part of the electron emission assembly, but also is a unique electrode having one potential relative to a ground potential outside the device, and only emits electrons to the space outside the device;
the electrode comprises various metals, alloys, carbon materials, or composite materials, or other materials for emitting electrons;
the electrode is in an acicular shape, a sharp shape, a threadlike shape, a clavate shape, a serrated shape, or a plate shape;
the electrode is designed into different structures and sizes according to the need; the electrode comprises a single or multiple electrodes with the same electric potential; and
the electrode has different potentials ranging from -1 Kv to -35Kv relative to the ground potential outside the air conditioning device according to different materials, shapes, structures, sizes and requirements of the electrode.

4. The environment-improving and healthful air conditioning device according to claim 1, wherein the electrons emitted by the electron emitter in the electron emission assembly are bound to oxygen molecules, carbon dioxide molecules, and water vapor molecules in air, or a molecular group composed of the molecules, or a nanoscale molecular group composed of the molecules with other molecules in air, so as to form negative electric nano particles distributed in corresponding air conditioning space;
corresponding electron emission assemblies are designed and distributed, and electron emitters with different quantities and the same potential are distributed according to the volume, shape, structure, size and requirement of the air conditioning space, and the structures and sizes of various air supply outlets, or scavenge ports, or ventilation grids, so that the density of the negative electric nano particles formed in the air conditioning space through the electrons emitted by the electron emitter is adjusted within a range more than 5 x 10³/cm³ but less than or equal to 10⁹/cm³ according to different use purposes and requirements, so that the corresponding air conditioning space is in an optimal health state.

5. The environment-improving and healthful air conditioning device according to claim 1, wherein due to the configuration of the electron emission assembly, the refrigerating system and/or the heating system, and the corresponding parts thereof can be removed from the air conditioning device as long as the parts matched with the electron emission assembly are combined again, so that the structure of the device is changed, and the volume and size of the entire device are reduced; although no temperature regulation is configured, the air conditioning device is still an environment-improving and healthful air conditioning device that not only reduces the cost, but also can expand the application of the device better and more easily; due to the configuration of the electron emission assembly, the refrigerating system or the heating system of the air conditioning device with the refrigerating system and the heating system is closed in case that the ambient temperature is suitable, and the air volume is adjusted according to the need, so that the air conditioning device is still an environment-improving and healthful air conditioning device that not only saves the energy consumption, but also greatly extends the working time of the air conditioning device, and the air conditioning device may be used at any time all the year round according to the need, which preferably serves for improving the environment and being healthful.

6. An application method of the environment-improving and healthful air conditioning device according to claim 1, wherein the electrons emitted by the electron emitter in the electron emission assembly form the negative electric nano particles with a certain density in the air conditioning space, the negative electric nano particles are distributed in the air conditioning space, and the air conditioning space is in a disinfection state; the negative electric nano particles in the air conditioning space can enter a human body through a respiratory system to provide lost electrons caused by the oxidative damage of DNA for the organism, so as to improve the immunity of organism and strengthen the health of a human body; the electrons and the negative electric nano particles formed by the electrons can quickly eliminate the damage to the human body caused by various contaminants, toxic materials, radioactive materials, and harmful nanoscale particles floating in air; so as to improve the dwelling environment and other special environments, and guarantee the successful performance of scientific research, industrial and agricultural production, and various works under special environment; and using the air conditioning device in various public places can prevent and control the spread of various respiratory system diseases and other diseases with infectiousness, and can eliminate the harmful germs and harmful substances in the air of production greenhouse and livestock shed in the production of agriculture and livestock breeding, optimize the breed, prevent and control the occurrence and spread of various diseases, and improve the yield.
